(19) Europäisches Patentamt
European Patent Office
Office européen des brevets

(11) Numéro de publication : **0 441 708 B1**

# (12) FASCICULE DE BREVET EUROPEEN

(45) Date de publication du fascicule du brevet : **03.08.94 Bulletin 94/31**

(51) Int. Cl.$^5$ : **C07C 45/67,** C07C 49/203, C07C 69/736

(21) Numéro de dépôt : **91400293.6**

(22) Date de dépôt : **07.02.91**

## (54) Procédé de préparation de cétones terpéniques.

(30) Priorité : **08.02.90 FR 9001441**

(43) Date de publication de la demande : **14.08.91 Bulletin 91/33**

(45) Mention de la délivrance du brevet : **03.08.94 Bulletin 94/31**

(84) Etats contractants désignés : **AT BE CH DE DK ES FR GB GR IT LI LU NL SE**

(56) Documents cités :
**EP-A- 0 044 771**
**EP-A- 0 118 354**
**DE-A- 2 350 194**
**CAN. J. CHEM., vol. 51, 1973, pages 848-849; S. WATANABE et al: "The new synthesis of 5,6-dimethyl-5-hepten-2-one from 2,3-dimethyl-1,3-butadiene"**

(56) Documents cités :
**TETRAHEDRON LETTERS, no. 38, 1978, pages 3575-3578; R. BAKER et al: "Reactions of active methylene and carbonyl compounds with myrcene catalysed by palladium and nickel complexes"**
**CHEMICAL ABSTRACTS, vol. 88, 1978, page 571, colonne 2, résumé no. 121457e,Columbus, Ohio, US; & JP-A-77 59 114**

(73) Titulaire : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy Les Lyon (FR)**
Inventeur : **Mercier, Claude**
**85 Avenue du Point du Jour**
**F-69005 Lyon (FR)**

(74) Mandataire : **Le Pennec, Magali et al**
**RHONE-POULENC RORER SA,**
**Direction des Brevets,**
**20 Avenue Raymond Aron**
**F-92165 Antony Cédex (FR)**

EP 0 441 708 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de cétones terpéniques. Elle a plus particulièrement pour objet la préparation de cétones terpéniques à partir de butadiène-1,3 substitué éventuellement sur le carbone 2 par une chaîne hydrocarbonée notamment polyénique et de préférence isoprénique.

Les cétones terpéniques polyéniques répondent à la formule générale suivante :

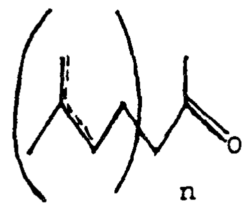

dans laquelle n est un nombre entier compris entre 1 et 4.

Il est connu par exemple selon le brevet EP 44 771 un procédé d'addition sur des diènes conjugués substitués, de préférence des butadiènes-1,3 substitués sur le carbone 2 par une chaîne hydrocarbonée, d'un composé ayant un atome de carbone activé. Les composés ayant un atome de carbone activé sont choisis à titre d'exemple parmi les aldéhydes, les cétones, les esters, les esters sulfoniques, les dérivés nitrés, les dérivés cyanés, les amides. Lors de la condensation de ces composés, ayant un atome de carbone activé, sur le butadiène substitué, on obtenait en fin de réaction un aldéhyde, une cétone, un ester, un dérivé nitré, un dérivé cyané ou une amide substitué par le radical butadiène qui sert de précurseur à des intermédiaires des vitamines A et E ou de parfums.

Can. J. Chem., 1973, 51, p. 848-849 décrit la préparation de méthyl-6 heptène-5 one-2 par la réaction de l'isoprène avec l'acétoacétate de méthyle, suivi par une décarboalcoxylation avec 5% NaOH.

La décarboxylation des β-cétoesters est décrite par exemple dans l'article de KRAPCHO et al, J. Org. Chem.,43, p. 143 et sequ. (1978) à l'aide d'un milieu composé d'eau et de diméthylsulfoxide. Cet article ne décrit pas la décarboxylation des β-cétoesters substitués par un groupe terpénique tel que l'isoprène. Il décrit l'utilisation d'un solvant tel que le diméthylsulfoxide que l'industrie cherche à éviter, car il est onéreux et dangereux.

Il est également connu selon l'article de J.M. DERFER et MM. DERFER (Kirk Othmer, 22, p. 731) de préparer la géranylacétone, cétone terpénique, par transestérification du linalol avec l'acétoacétate d'éthyle selon la réaction connu sous le nom de Caroll avec élimination de dioxyde de carbone. Cette réaction est spécifique du linalol. Le linalol étant une matière première onéreuse l'industrie cherche depuis longtemps un moyen d'accès aux vitamines évitant l'utilisation de ce composé.

La présente invention a permis d'atteindre cet objectif. Elle a pour objet un procédé de préparation de cétones terpéniques dans lequel on met en présence un polyène dérivé du butadiène-1,3 de formule:

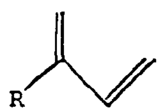

dans laquelle R représente un radical méthyl, polyénique et de préférence polyisoprénique avec un acétoacétate d'alkyle sur lequel on réalise une décarboalcoxylation avec de l'eau seule.

Le composé du butadiène 1,3 répond à la formule (I) suivante :

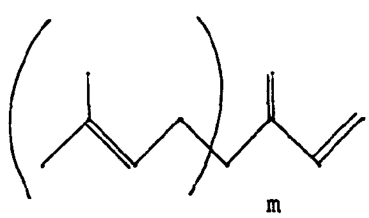

(I)

dans laquelle m est égal à un nombre entier égal ou supérieur à 0 et inférieur ou égal à 3.

On préfère parmi les composés de formule (I) utiliser:

- le myrcène
- l'isoprène
- le β-farnésène.

L'acétoacétate d'alkyle répond à la formule (II) suivante:

$$CH_3\text{-}CO\text{-}CH_2\text{-}CO_2\text{-}R_1$$

dans laquelle $R_1$ représente un radical hydrocarboné contenant 1 à 12 atomes de carbone de préférence sous forme d'une chaîne alkyle ou alkényle linéaire ou ramifiée ou d'un radical aromatique éventuellement substitué.

On préfère parmi l'ensemble des dérivés de formule (II) ceux pour lesquels $R_1$ représente un groupe alkyle contenant 1 à 4 atomes de carbone et tout particulièrement l'acétoacétate de méthyle.

La réaction de condensation est réalisée en présence d'un catalyseur à base de rhodium. Ce catalyseur est choisi parmi les sels, les oxydes, les complexes du rhodium en présence éventuellement d'un ligand. Parmi les agents ligands on peut citer les phosphines de formule (III) :

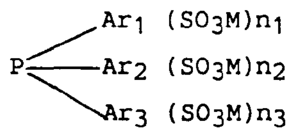

(III)

dans laquelle :

- $Ar_1$, $Ar_2$ et $Ar_3$ identiques ou différents représentent chacun un radical choisi parmi le groupe comprenant les radicaux phénylène et les radicaux naphtylène, ces radicaux étant éventuellement substitués.
- M est un reste cationique d'origine minérale ou organique choisi de manière que la phosphine de formule (III) soit soluble dans l'eau.
- $n_1$, $n_2$ et $n_3$ identiques ou différents sont des nombres entiers supérieurs ou égaux à 0 et inférieurs ou égaux à 3, l'un au moins étant supérieur ou égal à 1.

Le procédé selon l'invention est de préférence mis en oeuvre en utilisant au moins une phosphine de formule (III) dans laquelle $Ar_1$, $Ar_2$ et $Ar_3$ identiques ou différents représentent chacun un radical phénylène.

Encore plus préférentiellement, on utilise une phosphine soluble en milieu aqueux dans laquelle les groupements $SO_3M$ sont en position méta sur le noyau benzénique.

De préférence M est choisi parmi le groupe comprenant les cations dérivés des métaux Na, K, Ca, Ba, les ions $NH_4^+$ et ammonium quaternaire comme les ions tetraméthylammonium, tétrapropylammonium et tétrabutylammonium.

$n_1$, $n_2$ et $n_3$ sont de préférence égaux à 1.

On préfère utiliser la triphénylphosphine métatrisulfonée.

Selon un mode préférentiel de réalisation du procédé de l'invention le dérivé du rhodium est choisi parmi le groupe comprenant les sels inorganiques, organiques et les complexes du rhodium comme par exemple $RhCl_3$, $RhBr_3$, $Rh_2O$, $Rh_2O_3$, $Rh(NO_3)_3$, $Rh(CH_3COO)_3$, $Rh(CH_3COCHCOCH_3)_3$, $[RhCl(\text{cyclooctadiène-1,5})]_2$, $[RhCl(CO)_2]_2$, $RhCl_3(C_2H_5NH_2)_3$, $Rh_2(SO_4)_3$.

On préfère tout particulièrement utiliser $RhCl_3$ et $[RhCl(\text{cyclooctadiène-1,5})]_2$.

On utilise une quantité de rhodium ou de composé du rhodium telle que le nombre d'atome gramme de rhodium élémentaire par litre de solution réactionnelle soit compris entre $10^{-4}$ environ et 1 environ. De préférence il est compris entre 0,001 et 0,5 environ.

Pour une bonne mise en oeuvre du procédé, la quantité de phosphine est choisie de telle sorte que le nombre d'atomes gramme de phosphore trivalent rapporté à un atome gramme de rhodium soit compris entre 0,1 environ et 200 environ. De préférence, ce nombre est compris entre 3 environ et 100 environ.

La réaction d'addition peut être schématisée de la façon suivante :

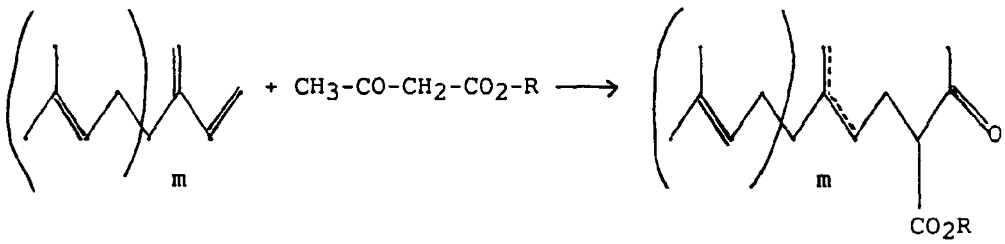

L'ester est mis en contact avec de l'eau de façon à effectuer une décarboalcoxylation. Il est tout à fait étonnant qu'en présence de composés de cette nature c'est à dire de β-cétoester polyénique il ne soit pas nécessaire d'adjoindre de solvant ou d'agent de décarboalcoxylation et que l'eau seule permette cette réaction en l'absence d'un quelconque catalyseur. Cela présente sur le plan industriel un avantage économique considérable, il n'y a production d'aucun produit secondaire difficile à éliminer. Les seuls sous produits de réaction sont le dioxyde de carbone et l'alcool de formule ROH qui sont faciles à éliminer.

L'ester utilisé pour effectuer la réaction de décarboalcoxylation peut être un produit purifié isolé ou le produit brut directement obtenu à l'étape de condensation entre le β-cétoester et le dérivé du butadiène. La décarboalcoxylation peut avantageusement être effectuée dans le même réacteur que la réaction d'addition.

La réaction de décarboalcoxylation est effectuée en phase vapeur ou en phase liquide à une température comprise entre 130°C et 500°C. Lorsque la réaction est effectuée en phase vapeur, la température est comprise de préférence entre 300 et 500°C et encore plus préférentiellement entre 350 et 400°C. Lorsque la réaction est réalisée en phase liquide, on préfère utiliser une température comprise entre 180 et 220°C. La réaction peut aussi être réalisée en phase ruisselante sur un lit de matériau solide non réactif tel que le quartz.

La réaction de décarboalcoxylation peut être effectuée à la pression atmosphérique, sous une pression inférieure ou sous une pression supérieure. On préfère néammoins travailler à une pression inférieure à 100bar et tout particulièrement à une pression comprise entre 1 et 20 bars. Les conditions de température et de pression seront adaptées par l'homme de l'art à la matière première utilisée.

Les dérivés cétoniques polyéniques obtenus sont des intermédiaires de synthèse important dans l'industrie des vitamines et des parfums.

On peut citer parmi ces composés :

- la géranylacétone,
- la méthylhepténone,
- la farnésylacétone.

La présente invention sera plus complètement décrite à l'aide des exemples suivants qui ne doivent pas être considérés comme limitatifs de l'invention.

Dans les exemples suivants, les formules (I) et (II) représentent les composés suivants :

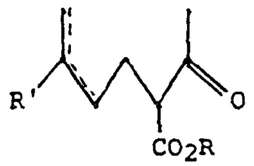

(I)

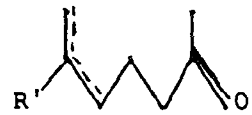

(II)

R = $CH_3$-  R' = 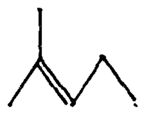  (Ia)  géranylacétone (IIa)

R = $CH_3$-  R' = $CH_3$  (Ib)  méthylhepténone (IIb)

R = 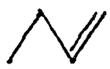  R' = 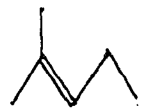  (Ic)

R = $C_2H_5$-  R' = $CH_3$  (Id)

EXEMPLE 1

Dans un autoclave en acier inoxydable préalablement purgé à l'azote , on introduit 150 mg de $[RhCl(cyclooctadiène\text{-}1,5)]_2$ (0,615 milliatome-gramme de rhodium), 4,2 g de triphénylphosphine trisulfonée sodique (6,2 milliatome-gramme de $P^{3+}$) 0,1 g de $Na_2CO_3$ (≈1millimole) et 70 ml d'eau.

On introduit ensuite 250 mmoles de myrcène à 99 % de pureté (34 g) et 300 millimoles d'acétylacétate de méthyle (34,8 g).

On chauffe sous agitation à 85°C durant 17 heures. Par chromatographie couche mince, on obtient un taux de transformation d'environ 100 % avec formation des β-cétoester (Ia) [mélange équimoléculaire (RMN$^1$H) exo-endo].

Le mélange biphasique (phase organique jaune pâle et phase aqueuse rouge orangée) est engagé dans l'étape de décarboxylation dans le même autoclave, on chauffe 3heures à 180°C, jusqu'à la fin de la montée en pression dûe au dégagement gazeux. La pression atteint 25 bar pour un réacteur de 750 ml. On refroidit, dégaze. L'analyse par chromatographie couche mince, gazeuse et RMN$^1$H après extraction du système biphasique donne un taux de transformation complet des β-cétoesters (Ia). On isole une phase organique contenant essentiellement les deux isomères de la géranylacétone IIa (exo et endo) : 40,3 g. La distillation permet d'isoler la géranylacétone (2 isomères) pure: 33 g ($Eb_1$ environ 82°C). Le rendement en géranylacétone distillée par rapport au myrcène engagé est de 67,4 %.

La phase aqueuse finale jaune orangée contient 0,54 g/l de rhodium soluble homogéne.

EXEMPLE 2

Dans un autoclave en acier inoxydable préalablement purgé à l'azote , on introduit 150 mg de $[RhCl(cyclooctadiène\text{-}1,5)]_2$ (0,615 milliatome-gramme de rhodium), 8,4 g de triphénylphosphine trisulfonée sodique (12,4 milliatome-gramme de $P^{3+}$) 0,1 g de $Na_2CO_3$ (≈1millimole) et 80 ml d'eau.

On introduit ensuite 250 mmoles de myrcène à 78 % de pureté (43,5 g) et 300 millimoles d'acétylacétate de méthyle (34,8 g).

On chauffe sous agitation à 91°C durant 17 heures. Par chromatographie couche mince de la phase organique, on obtient un taux de transformation d'environ 100 % sur le myrcène, avec formation des β-cétoester (Ia) [mélange équimoléculaire (RMN$^1$H) endo,exo].

Le mélange biphasique (phase organique jaune pâle et phase aqueuse rouge orangée) est engagé dans l'étape de décarboxylation dans le même autoclave, on chauffe 3heures à 180°C, jusqu'à la fin de la montée

en pression dûe au dégagement gazeux. On refroidit, dégaze. L'analyse par chromatographie couche mince (CCM), gazeuse (CPV) et RMN$^1$H après extraction du système biphasique donne un taux de transformation complet des β-cétoesters (Ia). On isole une phase organique contenant essentiellement les deux isomères de la géranylacétone (IIa) (exo et endo): 48,7 g. La distillation permet d'isoler une première fraction ($Eb_1$ environ 25-30°C) contenant les hydrocarbures C10 inertes contenus dans le myrcène technique (9,4 g) et une deuxième fraction correspondant à la géranylacétone (2 isomères) pure: 30,6 g ($Eb_1$ environ 82°C). Le rendement en géranylacétone distillée est de 63 %.

La phase aqueuse finale jaune orangée contient le rhodium soluble homogéne.

EXEMPLE 3

Dans un réacteur tubulaire de 18 mm de diamètre, on dispose 10 ml de poudre de quartz que l'on préchauffe 30 mn à 350°C sous courant d'azote (3,5 l/h). Au moyen d'un pousse seringue, on injecte alors le β-cétoester Ia (mélange des 2 isomères pureté de 95 %) à un débit liquide de 10mlh$^{-1}$ et l'eau permutée par un deuxième pousse seringue à 3,5mlh$^{-1}$ (environ 5 équivalents par rapport au β-cétoester) soit un temps de contact moyen de 3,8 secondes. Après 35 minutes de réaction en phase vapeur, l'analyse du condensat biphasique (phase aqueuse incolore, phase organique jaune) par chromatographie couche mince, gazeuse et RMN$^1$H montre la formation de géranylacétone avec un taux de transformation du β-cétoester de 70 %, une sélectivité en géranylacétone de 85 % et une productivité de 1,1kgh$^{-1}$l$^{-1}$.

EXEMPLE 4

Dans un tricol de 100ml, surmonté d'une colonne de distillation et muni d'un thermomètre, on charge sous azote 10 g du β-cétoester (Ia) (45 % endo et 55 % exo), chauffe rapidement la masse réactionnelle sous agitation magnétique à 170-180°C et introduit alors, à l'aide d'un pousse seringue à raison de 1,2ml/h l'eau dans la masse réactionnelle. On suit l'évolution de la réaction par chromatographie en phase gazeuse sur des aliquots et par dégagement de $CO_2$. Après 1 h 30 de réaction, on observe un taux de transformation complet et un rendement en géranylacétone de 92 % après distillation.

EXEMPLE 5

Dans l'appareillage décrit à l'exemple 4, on charge sous azote 10 g du β-cétoester (Ib) (40 % endo et 60 % exo) et sur la masse réactionnelle, portée à 180°C, on injecte en 2 heures, 2,5 ml d'eau permutée. On obtient un taux de transformation complet analysé par chromatographie phase gazeuse et RMN$^1$H et après distillation (point d'ébullition à 15 mm de mercure 69°C) on recueille 6,1 g de méthylhepténone (IIb) (rendement = 89,7 %) identifiée par chromatographie phase gazeuse, RMN$^1$ et infrarouge.

EXEMPLE 6

Avec l'appareillage décrit dans l'exemple 3, on opère à 400°C sous courant d'azote (3,5 lh$^{-1}$ en CNTP). Au moyen d'un pousse seringue, on injecte le β-cétoester (Ia) (pureté 96 % - mélange des deux isomères endo : 55/exo 45) à un débit liquide de 10 mlh$^{-1}$ (36,5 millimoles h$^{-1}$) et simultanément l'eau permutée au moyen d'un deuxième pousse seringue à 2,4 mlh$^{-1}$ (133,3 millimoles h$^{-1}$ soit 3,65 équivalents) correspondant à un temps de contact moyen de 4,6 secondes. Après 40 mn de réaction en phase vapeur, l'analyse du condensat biphasique (1,3 ml de phase aqueuse incolore et 3,90 g de phase organique jaune après élimination des légers au rotavap) montre par CCM, CPV et RMN$^1$H la formation de géranylacétone (IIa) avec un taux de transformation du β-cétoester (Ia) d'environ 84 % et une sélectivité en géranylacétone de 85 %.

EXEMPLE 7

Selon l'exemple 6, avec l'appareillage décrit dans l'exemple 3, mais en opérant à 450°C, on obtient après 35 mn de réaction un condensat biphasique (1,2 ml de phase aqueuse incolore et 4,38 g de phase organique jaune dont l'analyse CCM, CPV et RMN$^1$H montre la formation de géranylacétone (IIa) avec un taux de transformation du β-cétoester (Ia) d'environ 63 % et une sélectivité en géranylacétone de 73 %.

EXEMPLE 8

On reproduit l'exemple 6, à 400°C, mais en modifiant le débit d'injection de β-cétoester à 4,95 mlh$^{-1}$ (18,2

millimole $h^{-1}$) tout étant égal par ailleurs, soit un rapport de 7,3 équivalents d'eau par rapport au β-cétoester et un temps de contact moyen de 4,8 secondes. Après 1 heure 05 de réaction, on obtient un condensat biphasique contenant 2,2 ml de phase inférieure aqueuse incolore et 3,52 g de phase organique (après élimination des légers au rotavap). L'analyse de cette phase organique (CCM, CPV et $RMN^1H$) indique un taux de transformation complet sur le β-cétoester (Ia) et formation de la géranylcétone (IIa) avec une sélectivité de 75 % et une productivité de 500 g/l de réacteur et par heure.

EXEMPLE 9

Avec l'appareillage décrit dans l'exemple 3, mais à 250°C, on opère alors selon la technique du lit fixe ruisselant (cf. Technique de l'Ingénieur, Vol. J4, "Génie Chimique", 1965, paragraphe 2-4).

On injecte sous azote à 3,5 $lh^{-1}$, le β-cétoester (Ia) de l'exemple 6 à un débit liquide de 4,95 $mlh^{-1}$ (18,2 millimoles $h^{-1}$) et simultanément l'eau permutée au moyen d'un deuxième pousse seringue à 2,4 $mlh^{-1}$ liquide (133,3 millimoles soit 7,3 équivalents).

Après 55 mn de réaction, on recueille un condensat biphasique avec 1,9 ml de phase aqueuse incolore et 3,93 g de phase organique jaune pale (après élimination des légers au rotavap) dont l'analyse CCM, CPV et $RMN^1H$ indique un taux de transformation du β-cétoester d'environ 25 % et formation de la géranylacétone (IIa) avec une sélectivité de 88 %.

EXEMPLE 10

On reproduit l'exemple 4 mais on effectue la réaction dans un autoclave en acier inox, SOTELEM de 300 ml équipé d'une turbine de RUCHTON permettant un excellent transfert, à pression atmosphérique, chauffé à 200°C avec injection de l'eau dans la masse réactionnelle au moyen d'un pousse seringue RAZEL, on recueille le méthanol dans un piège après distillation et utilise un compteur à gaz hydraulique de précision pour comptabiliser le volume de $CO_2$ dégagé.

Sous azote, on charge 131,25 g (0,5 mole) de β-cétoester de l'exemple 6 (96 % de pureté), on chauffe à 200°C en agitant à 2000 tours/mn et injecte alors l'eau permutée à 1 $mlh^{-1}$ et contrôle l'avancement de la réaction par le volume de $CO_2$ dégagé.

Après 1 heure 05, on a injecté 15,5 ml d'eau (1,72 équivalent par rapport au β-cétoester introduit) et dégagé 11,5 l de $CO_2$ lorsque ce dégagement cesse. Après refroidissement, on isole 97,2 g (115 ml) de masse réactionnelle dont l'analyse (CCM, CPV et $RMN^1H$) indique un taux de transformation complet du β-cétoester (Ia) en rendement en géranylacétone (IIa) de 94 % et une pureté sur le brut de 95 %.

Le distillat incolore de 24 ml contient majoritairement le méthanol (contrôlé par CPV) et l'eau excédentaire ce qui permet par simple distillation une récupération aisée du méthanol.

EXEMPLE 11

Dans un autoclave en acier inoxydable de 125 ml préalablement purgé à l'azote, on introduit 38,1 millimoles de β-cétoester Ia (10 g - pureté 96 %), 111,1 millimoles d'eau (2 g) c'est-à-dire 2,92 équivalents par rapport au β-cétoester. On chauffe alors à 220°C, sous agitation, jusqu'à la fin de la montée en pression due au dégagement gazeux c'est-à-dire 0,5 heure et la pression atteint 24 bar.

On refroidit, dégaze et l'analyse après décantation du système biphasique donne par CCM, CPV et $RMN^1H$ un taux de transformation complet sur le β-cétoester et une phase organique contenant essentiellement les deux isomères de la géranylacétone (IIa) obtenus avec un rendement de 91 % et une pureté de 93 %.

EXEMPLE 12 A 16

On opère comme dans l'exemple 11 mais à une température variable.

Le tableau ci-dessous résume les résultats :

| N° EXEMPLE | TEMPERATURE REACTION (°C) | DUREE REACTION | PRESSION MAXIMALE (bar) | RESULTATS TT(ßCE) | RT(GA) |
|---|---|---|---|---|---|
| 12 | 180°C | 2 H 00 | 19 bar | 100 | 91 |
| 13 | 150°C | 3 H | 5 bar | 34 | 95 |
| 14 | 250°C | 30 mn | 25 bar | 100 | 90 |
| 15 | 295°C | 30 mn | 29 bar | 100 | 89 |
| 16 | 165°C | 6 H 00 | 11 bar | 100 | 91 |

EXEMPLE 17

Dans un autoclave en acier inoxydable de 125 ml, préalablement purgé à l'azote, on introduit 21,5 millimoles de β-cétoester suivant:

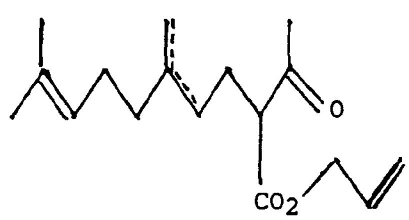

(Ic)

(6,8 g - Pureté environ 90 %) et 3 équivalents d'eau par rapport au β-cétoester. On chauffe alors à 180°C durant 3 heures 30 (fin de la montée en pression). Après refroidissement et dégazage, l'analyse après décantation du système biphasique donne par CPV, CCM et $RMN^1H$, un taux de transformation de 97 % sur le β-cétoester allylique (Ic) et formation de la géranylacétone (IIa) avec un rendement de 89 % et une pureté de 91 %.

EXEMPLE 18

On reprend l'exemple 12 du brevet FR 2.486.525 de la demanderesse d'addition sur l'isoprène de l'acétylacétate d'éthyle mais on isole pas le β-cétoester (Id) et on enchaîne directement la décarboéthoxylation dans le même autoclave ce qui permet un "one pot" d'isoprène à la méthylhepténone sans isoler les intermédiaires.

De manière plus précise, dans un autoclave en acier inoxydable de 125 ml, préalablement purgé à l'argon, on introduit 34 mg de $[RhCl(cyclooctadiène\text{-}1,5)]_2$ (0,14 milliatome-gramme de rhodium), 0,3 g de triphénylphosphine trisulfonée sodique (0,44 milliatome-gramme de $P^{3+}$), 80 mg de $Na_2CO_3$ (0,73 millimole) et 30 $cm^3$ d'eau. On introduit ensuite 102 millimoles d'isoprène (6,9 g) et 125 millimoles d'acétylacétate d'éthyle (16,2 g). On chauffe sous agitation à 100°C pendant 4 heures puis enchaîne directement par 3 heures à 220°C (la pression s'élève à 37 bar). Après refroidissement, dégazage et décantation du système biphasique, l'analyse (CPV, CCM et $RMN^1H$) de la phase organique donne : un taux de transformation complet sur l'isoprène et formation de méthylhepténone (I9
Ib) avec un rendement de 71 % (ex : isoprène).

EXEMPLE 19

L'appareillage est constitué d'un ballon tricol de 100 ml avec agitation magnétique, colonne monobloc vigreux, ballon récepteur relié à une cuve à eau surmontée d'une éprouvette graduée pour mesurer le volume de $CO_2$ dégagé, pousse seringue RAZEL avec aiguille plongeante inox pour l'introduction d'eau et chauffage par bain d'huile régulé.

On charge, sous azote, dans le ballon 38,1 millimoles de β-cétoester Ia (10 g - pureté 96 %), chauffe rapidement la masse réactionnelle sous agitation magnétique à 160°C et introduit alors à l'aide d'un pousse seringue à raison de 1,2 $mlh^{-1}$ l'eau permutée dans la masse réactionelle. On suit l'évolution de la réaction par dégagement du $CO_2$. Après 1 heure 30, 600 ml de $CO_2$ sont formés et on note par analyse CPV, $RMN^1H$ et CCM un taux de transformation de 60 % sur le β-cétoester et formation de la géranylacétone avec un rendement de 95 %.

EXEMPLE 20 ET 21

On opère comme dans l'exemple 19 mais à température variable. Le tableau ci-dessous résume les résultats :

| N° EXEMPLE | TEMPERATURE REACTION (°C) | DUREE REACTION | RESULTATS (*) TT(ßCE) | RT(GA) |
|---|---|---|---|---|
| 20 | 200°C | 50 mn | 100 | 93 |
| 21 | 220°C | 1 H | 100 | 91 |

(*) T.T(ßCE) : Taux de transformation du ß-cétoester (Ia)

R.T(GA) : Rendement en géranylacétone (IIa)

EXEMPLE 22

L'exemple suivant illustre comment le procédé peut être mis en oeuvre avantageusement pour produire la géranylacétone au départ de matières premières commerciales comme le Myrcène Technique (Pureté environ 75 à 80 %). A la fin de l'étape de condensation de l'acétoacétate de méthyle sur le myrcène technique selon le brevet FR 2.486.525 de la demanderesse, on peut, au lieu de décarbométhoxyler (directement) comme illustré dans l'exemple 4, opérer de la manière suivante:

Après arrêt de la réaction, on refroidit à température ambiante. Le contenu du réacteur est soutiré et on isole ensuite le produit de la réaction qui est en phase organique en séparant cette dernière de la phase aqueuse contenant le catalyseur par décantation et éventuellement par une extraction à l'aide d'un solvant convenable. La solution aqueuse peut être recyclée dans le réacteur pour catalyser une nouvelle réaction. La solution aqueuse peut aussi rester dans le réacteur, les produits organiques étant alors soutirés après décantation.

La phase organique ainsi recueillie constitue le β-cétoester brut et contient (au départ d'un myrcène technique à 78,2 % de pureté en myrcène) 71,2 % de β-cétoester, accompagné essentiellement des inertes du myrcène technique (comme le limonène), de l'excès d'acétoacétate de méthyle et d'environ 1 % d'eau.

On opère comme dans l'exemple 4 mais en chargeant 10 g de β-cétoester brut à 71,2 % en pureté en β-cétoester (soit 28,25 millimoles). Après 1 heure de réaction à 210°C, le dégagement de $CO_2$ cesse.

L'analyse du brut réactionnel indique un taux de transformation complet sur le β-cétoester et un rendement en géranylacétone de 92 % et une pureté de 79 %.

On note que le distillat contient les inertes du myrcène technique (limonène : $Eb_{760}$ environ 172°C), l'excès

d'acétoacétate de méthyle en plus du méthanol formé.

## Revendications

1. Procédé de préparation de cétones terpéniques caractérisé en ce que dans une première étape on fait réagir un polyène de formule :

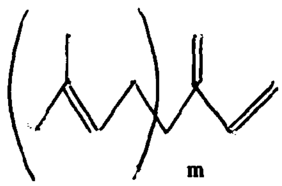

(I)

dans laquelle m est égal ou supérieur à 0 et égal ou inférieur à 3 avec un β cétoester puis dans une deuxième étape on effectue une décarboalcoxylation avec de l'eau seule.

2. Procédé de préparation de cétones terpéniques selon la revendication 1 caractérisé en ce que le β cétoester répond à la formule (II) suivante ::

$$CH_3\text{-}CO\text{-}CH_2\text{-}CO_2\text{-}R_1 \qquad (II)$$

dans laquelle $R_1$ représente un radical hydrocarboné contenant 1 à 12 atomes de carbone,

3. Procédé selon la revendication 1 caractérisé en ce que dans une première étape on fait réagir le myrcène avec l'acétoacétate de méthyle ; dans une deuxième étape on décarboalkoxyle le composé obtenu à la première étape.

4. Procédé selon la revendication 1 caractérisé en ce que la deuxième étape est réalisée à une température comprise entre 130 et 500°C et de préférence entre 180 et 220°C lorsque la réaction est réalisée en phase liquide et entre 350 et 400°C lorsqu'elle est réalisée en phase vapeur.

5. Procédé selon la revendication 2 caractérisé en ce que la deuxième étape est réalisée à une pression inférieure ou égale à 100 bar et de préférence entre 1 et 20 bar.

6. Procédé selon l'une quelconque des revendications précédentes caractérisé en ce que les deux étapes sont réalisées dans le même réacteur.

## Patentansprüche

1. Verfahren zur Herstellung terpenischer Ketone, dadurch gekennzeichnet, daß man in einer ersten Stufe ein Polyen der Formel:

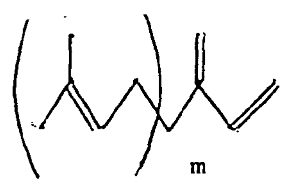

(I)

worin m gleich oder größer als 0 und gleich oder niedriger als 3 ist, mit einem β-Ketoester umsetzt und anschließend in einer zweiten Stufe eine Decarbalkoxylierung allein mit Wasser durchführt.

2. Verfahren zur Herstellung von terpenischen Ketonen gemäß Anspruch 1, dadurch gekennzeichnet, daß der β-Ketoester der folgenden Formel (II):

$$CH_3\text{-}CO\text{-}CH_2\text{-}CO_2\text{-}R_1 \qquad (II)$$

entspricht, worin $R_1$ für einen Kohlenwasserstoffrest mit 1 bis 12 Kohlenstoffatomen steht.

3. Verfahren gemäß Anspruch 1, dadurch gekennzeichnet, daß man in einer ersten Stufe Myrcen mit Methylacetoacetat umsetzt; und in einer zweiten Stufe die bei der ersten Stufe erhaltene Verbindung decarbalkoxyliert.

4. Verfahren gemäß Anspruch 1, dadurch gekennzeichnet, daß man die zweite Stufe bei einer Temperatur zwischen 130 und 500°C und vorzugsweise zwischen 180 und 220°C, wenn die Reaktion in flüssiger Phase erfolgt, und zwischen 350 und 400°C, wenn die Reaktion in der Dampfphase erfolgt, durchführt.

5. Verfahren gemäß Anspruch 2, dadurch gekennzeichnet, daß die zweite Stufe bei einem Druck von niedriger als oder gleich 100 bar und vorzugsweise zwischen 1 und 20 bar durchgeführt wird.

6. Verfahren gemäß einem der vorhergehenden Ansprüche, dadurch gekennzeichnet, daß die beiden Stufen in dem gleichen Reaktor durchgeführt werden.

## Claims

1. Process for the preparation of terpenic ketones, characterized in that, in a first stage, a polyene of the formula

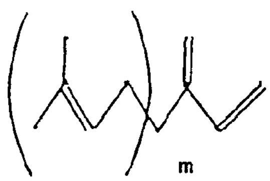

(I)

in which m is equal to or greater than 0 and equal to or smaller than 3, is reacted with a β-keto ester, and then, in a second stage, a decarbalkoxylation with only water is carried out.

2. Process for the preparation of terpenic ketones according to Claim 1, characterized in that the β-keto ester corresponds to the formula (II) below:

$$CH_3\text{-}CO\text{-}CH_2\text{-}CO_2\text{-}R_1 \qquad (II)$$

in which $R_1$ represents a hydrocarbon radical having 1 to 12 carbon atoms.

3. Process according to claim 1, characterized in that, in a first stage, myrcene is reacted with methyl acetoacetate and, in a second stage, the compound obtained in the first stage is decarbalkoxylated.

4. Process according to claim 1, characterized in that the second stage is carried out at a temperature of between 130 and 500°C and preferably between 180 and 220°C if the reaction is carried out in the liquid phase, and between 350 and 400°C if it is carried out in the vapour phase.

5. Process according to claim 2, characterized in that the second stage is carried out under a pressure of less than or equal to 100 bar and preferably between 1 and 20 bar.

6. Process according to any one of the preceding claims, characterized in that the two stages are carried out in one and the same reactor.